Europäisches Patentamt

European Patent Office

Office européen des brevets

(11) Publication number: **0 261 616**
**A2**

(12) # EUROPEAN PATENT APPLICATION

(21) Application number: 87113727.9

(22) Date of filing: 19.09.87

(51) Int. Cl.4: **C08H 1/00** , C09H 7/00 , A61K 47/00

(30) Priority: 25.09.86 US 911405

(43) Date of publication of application:
30.03.88 Bulletin 88/13

(84) Designated Contracting States:
CH DE FR LI

(71) Applicant: BASF Corporation
9 Campus Drive
Parsippany, NJ 07054(US)

(72) Inventor: Finnan, Jeffrey Lawrence
13689 Phelps
Southgate Michigan 48195(US)
Inventor: Lisa, Rudolph Ernest
28330 Elba Drive
Grosse Ile Michigan 48138(US)
Inventor: Chaundy, Frederick Kenneth
28731 South Point Drive
Grosse Ile Michigan 48138(US)

(74) Representative: Höller, Klaus, Dr. et al
BASF Aktiengesellschaft Carl-Bosch-Strasse 38
D-6700 Ludwigshafen(DE)

(54) **Method for rapid crosslinking of a protein.**

(57) The present invention relates to a method for the rapid crosslinking of a protein. More particularly, the protein is gelatin. The gelatin is crosslinked by combining a water miscible alcohol and acetaldehyde to the gelatin so that a moist intimate mixture results. Preferably, a mixture of gelatin, alcohol, and an oil-soluble vitamin (e.g. vitamin A) is crosslinked by the addition of acetaldehyde. The crosslinking of gelatin serves to stabilze the vitamin during conditions of high shearstress, as found in pelleting and extrusion operations involved in food and feed proecessing. Most preferably, a spray-congealed mixture of gelatin, vitamin A oil, sorbitol, and water are subjected to acetaldehyde vapors so that rapikd crosslinking of the gelatin occurs.

EP 0 261 616 A2

## METHOD FOR RAPID CROSSLINKING OF A PROTEIN

The present invention relates to a method for the rapid crosslinking of a protein. More particularly, the protein is gelatin. The gelatin is crosslinked by combining a water-miscible alcohol and acetaldehyde to the gelatin so that a moist intimate mixture results. Preferably, a mixture of gelating, alcohol, and an oil-soluble vitamin (e.g. vitamin A) is cross-linked by the addition of acetaldehyde. The crosslinking of gelatin serves to stabilize the vitamin during conditions of high shear-stress, as found in pelleting and extrusion operations involved in food and feed processing. Most preferably, a spray-congealed mixture of gelatin, vitamin A oil, sorbitol, and water are subjected to acetaldehyde vapors so that rapid crosslinking of the gelatin occurs.

The closest art of which the inventors are aware are: (1) U.S. 2,172,300; (2) U.S. 3,028,308; and (3) U.S. 3,232,761.

U.S. Patent 2,172,300 teaches the the use of acetals for hardening photographic gelatin layers. It is well recognized that with respect to gelatin, the term "hardening" is equivalent to crosslinking. Thus, U.S. 2,172,300 teaches the crosslinking of gelatin with acetals. It is also well known that alcohols combine with aldehydes to form acetals. (See Organic Chemistry, R.T. Morrison and R.N. Boyd, second edition, copyright 1966, Allyn and Bacon, Inc..page 641). However, even though the present invention utilizes the combination of water-miscible alcohol and acetaldehyde, it has been found that upon substituting a corresponding acetal of acetaldehyde, the speedy crosslinking reaction characterizing the present invention does not occur. Rather, the use of the acetal results in a significantly slower crosslinking reaction. These results are illustrated in Example II below.

U.S. 3,028,308 (hereinafter '308 patent) teaches the use of a dialdehyde (glyoxal ) for crosslinking gelatin in order to make time-released formulations. Futhermore, these formulations may comprise vitamins. However, this patent does not teach the use of an alcohol as a reactive species in the crosslinking reaction itself. Although the use of an "...alcoholic (90 percent) solution of methocel HG-60..." and "Additional anhydrous ethanol was added..." are referred to Examples 4, 5, and 6 of the '308 patent, it is clear that the alcohol plays no reactive role in the crosslinking reaction described in the '308 patent. Rather, the crosslinking reaction was completed in Example 1 (which utilized no alcohol), and the crosslinked product produced in Example 1 is utilized in Examples 4, 5, and 6. Thus the '308 patent differs from the present invention in that alcohol is not utilized in the crosslinking reaction.

U.S. 3,232,761 (hereinafter '761 patent) relates to the processing of photographic emulsion layers. In particular, this patent teaches the hardening of an emulsion layer comprising gelatin. Dialdehydes are used as the hardening agents, specifically glutaraldehyde, beta-methyl glutaraldehyde, etc. However, the alcohols used in the '761 patent are aromatic alcohols which are not water-miscible. It is believed that the aromatic alcohols utilized in the '761 patent are incapable of producing the rapid cross-linking found in the present invention.

The present invention is concerned with a method of rapidly crosslinking a protein, the method being carried out by combining a protein, a water-miscible alcohol, and acetaldehyde, so that a moist intimate mixture results. Preferably, the protein is gelatin. Preferably, the moist, intimate mixture further comprises a fat-soluble vitamin, such as vitamin A or vitamin E. The intimate mixture of gelatin, fat-soluble vitamin, and water-miscible alcohol is preferably spray-congealed, after whlich the spray-congealed product is then exposed to acetaldehyde, the acetaldehyde most preferably being in the vapor state.

It is an object of the present invention to rapidly crosslink a protein.

It is a further object of the present invention to rapidly crosslink gelatin.

It is a further object of the present invention to rapidly crosslink gelatin which is in intimate admixture with a fat-soluble vitamin and a water-miscible alcohol.

It is a further object of the present invention to rapidly crosslink protein within spray-congealed powder particles each comprising gelatin, fat-soluble vitamin, and water-miscible alcohol.

It is a further object of the present invention to rapidly crosslink gelatin found within a spray-congealed powder by subjecting the spray-congealed powder to acetaldehyde vapors

It is a further object of the present invention to enable the rapid crosslinking of protein with acetaldehyde.

It is a further object of the present invention to impart shear-stress stability to beadlets containing fatsoluble vitamins which are subjected to a high-moisture, high-shear, and high temperature environment for a brief period of time.

The present invention is concerned with the rapid crosslinking of proteins with acetaldehyde. The term "rapid" is obviously relative to prior art reaction rates. It is well known that crosslinking of proteins occurs much more quickly with formaldehyde than with acetaldehyde. However, it is preferable to crosslink with acetaldehyde if the resulting product is to be used for edible purposes. The prior art means of crosslinking with acetaldehyde have indicated that crosslinking requires approximately a two week period (see U.S. Patent 3,027,300). However, it has been unexpectedly found that significant crosslinking of proteins can be achieved in a period of as little as 15 minutes at temperatures between 50° and 60°. It has been found that the addition of a water-miscible alcohol together with acetaldehyde results in a rapid crosslinking reaction. In general, significant crosslinking occurs in three hours utilizing this process, and significant crosslinking most often occurs within a time period of 15 minutes to three hours, depending upon conditions. Thus, the phrase "rapid crosslinking" designates significant crosslinking (i.e., the formation of a gel which is insoluble in boiling water) in a time period less than three hours, generally 15 minutes to three hours.

In carrying out the process of the present invention, it is necessary to form a "intimate mixture" comprising the protein, the alcohol, and the acetaldehyde. The term "intimate mixture" is most preferably a "complete mixture," i.e., a uniform dispersion of molecules of the alcohol, the acetaldehyde, and the protein. However, other degrees of intermixture are intended to be included in the term "intimate mixture." For example, an emulsion wherein the diameter of the droplets (e.g., the droplets of vitamin A oil) is less than 10 microns is considered to be intimately mixed with the liquid (e.g., solution of alcohol, protein and acetaldehyde) within which the droplets are emulsified. Likewise, a relatively dry powder (the powder particles having a diameter between 100 and 800 microns), the powder particles each containing protein, vitamin A and alcohol is considered to be in an "intimate mixture" with acetaldehyde if exposed to acetaldehyde in either the liquid phase or the vapor phase. Thus, the creation of an "intimate mixture" leads to a high degree of accessibility of each of the reactive species with respect to one another. Examples of the means of obtaining a "intimate mixture" include, but are not limited to: dissolving, microencapsulating, spray drying, spray congealing, spray formulating, emulsifying, reduction to powder etc.

It has been found that the intimate mixture must be "moist" in order for the crosslinking reaction to occur. As can be seen in Example 1, the reaction can be carried out under virtually "dry" conditions. However, the spray-congealed powder of Example 1 contained approximately 3 percent moisture. It is believed that a minimum of 3 percent moisture is necessary for carrying out the process of the present invention. However, the crosslinking reaction may be carried out while the mixture has 40% moisture, or even more. The water is believed to supply a solvating/hydrating function. In addition, the water is believed to act as a catalyst for the crosslinking reaction. In relatively dry powders which contains vitamin A beadlets, the low levels of water probably aid the transport of acetaldehyde into the interior of the beadlets.

The process of the present invention requires the presence of a water-miscible alcohol. The water-miscible alcohols of use in the invention can have from 1 to 8 hydroxyl groups. Thus alcohols from methanol to sucrose are applicable in the present invention. Most preferably the alcohol is sorbitol, a polyhydroxy alcohol. Other alcohols which are preferred include glycerol, propylene glycol, mannitol, ethanol, and methanol, among others. The use of alcohols which are not water soluble, such as aromatic alcohols, are not believed to be operable in the process of the present invention, as they are not believed to allow the crosslinking reaction to occur due to their insolubility in water and/or steric problems created by the bulk of the aromatic molecule. For steric reasons, highly mobile water miscible alcohols are preferred to more bulky water miscible alcohols.

It has been found that if carrying out the process of the present invention with all ingredients in the liquid phase while the ingredients are exposed to the atmosphere, it is preferred to first allow the alcohol and acetaldehyde to react for a short period (e.g., about ten minutes), following which this reaction product is added to a warm aqueous solution of the protein. This procedure is described in Examples 3 and 4. It has been theorized that the alcohol and acetaldehyde react to form a hemiacetal which is suitable for obtaining the desired crosslinking reaction of the present invention. However, it is not known whether a hemiacetal forms upon the admixture of alcohol and acetaldehyde, but it has been believed that the formation of the hemiacetal or some other species (other than the acetal) is likely before the initiation of the crosslinking reaction. However, it is known that the reaction between the alcohol and the acetaldehyde can form a hemiacetal, which is believed to be effective in carrying out the process of the present invention. Thus an "intimate mixture of protein, water-miscible alcohol, and acetaldehyde" is meant to comprise the addition of a hemiacetal of acetaldehyde to the protein. However, as can be seen in Example 5 (Comparative), the acetal of acetaldehyde (referred to as acetaldehyde diethyl acetal) is not operable in the process of the present invention and is not considered to be within the phrase "intimate mixture of protein, alcohol, and acetaldehyde."

In the most preferred embodiments of the present invention, gelatin is utilized as the protein. Furthermore, in this process a fat-soluble vitamin is mixed into the gelatin, this vitamin most preferably being vitamin A. It is preferred to first make an intimate mixture of water, gelatin, vitamin A oil, and sorbitol, following which this mixture is spray congealed so that a powder having a particle diameter of approximately 100-800 microns results. The powder is dried (to about 5% of moisture) in a fluidized bed. The air entering the fluidized bed has a temperature between 50°C and 60°C. The powder is then placed in a closed vessel which after evacuation is heated to a temperature between 60° and 90°, after which acetaldehyde vapors are circulated through the powder for approximately three hours. Most preferably acetaldehyde is added in a quantity sufficient to achieve a pressure between 5 psig and 30 psig. The acetaldehyde vapors are intimately admixed with the powder by virtue of the small size of the powder particles and the high mobility and permeability of the vapor. The crosslinking reaction is virtually complete in the three hour period. At this point the crosslinked vitamin A-containing powder may be incorporated into feed formulations under conditions of heat, moisture, and high shear. After incorporation, the feedstock is generally pelleted or extruded under hot, moist and high shear-stress conditions. The difference in feedstock retention of vitamin A for crosslinked and non-crosslinked products, made by an extrusion process, is as follows:

65 - 70 percent retention of vitamin A after extrusion for NON-CROSSLINKED powder; and

90 - 99 percent retention of vitamin A after extrusion for powder which was CROSSLINKED by the process described above.

In general, it is believed that the amounts of the ingredients utilized in the process are as follows: 3-50 parts water, 5-50 parts gelatin, 10-30 parts water-miscible alcohol, and 0.5-15 parts of acetaldehyde. Preferably an intimate mixture of gelatin, acetaldehyde, and alcohol is produced by spray-congealing the gelatin and alcohol followed by exposure to acetaldehyde vapors. This preferred product comprises between 3-10 parts water, 25-50 parts gelatin, 15-40 parts alcohol, and 3-10 parts acetaldehyde.

Furthermore, it is believed that the process of the present invention is operable within the temperature range of 20°C to 150°C. However, the preferred temperature range is 50°C to 60°C (during the drying process) and 60°C to 90°C (during the crosslinking reaction).

Example 1

The following ingredients were utilized in the making of an aqueous emulsion:
(a) Sorbitol        23 parts
(b) Unhydrolyzed Gelatin        36 parts
(c) Vitamin A Oil -    41 parts
(2.1 million iu/gm, containing 80 mg of ethoxyquin per million iu of vitamin A; iu stands for "international units")
These percentages are given on a dry weight basis. The emulsion was made by adding these ingredients to 81.8 parts of water, on a weight basis. The emulsion was created by homogenizing the above described mixture until the resulting oil droplets were approximately 2 microns in diameter. The emulsion was then spray congealed (using 78 parts of hydrophobic starch as the absorbent). The spray congealing process was carried out as described in U.S. 3,445,463, in particular column 1, lines 40-47. One hundred grams of the resulting powder (the powder had an enzyme method assay of about 676,000 iu/gm, 23 percent starch, and 5.6 percent moisture) were placed in a 250 milliliter closed vessel. The vessel was evacuated and four grams of acetaldehyde vapor were injected into the vessel through a rubber septum. The vessel was placed in a convective oven at 90°C for a period of two hours, the vessel being removed every 15 minutes for shaking. It was noted that the maximum pressure achieved in the vessel was about 10 psig. After two hours, the sample was removed from the vessel and placed in a small fluid bed. Room temperature nitrogen was used to fluidize the powder for two hours in order to remove the residual acetaldehyde. The resulting powder was assayed again by the enzyme method, and the powder showed a value of only 39,750 iu/gm, indicating significant crosslinking. It has also been found that the crosslinking does not limit the bioavailability of ingredients.

### Example II (Comparative)

A process was carried out as described in Example 1 except that in place of sorbitol, an equal weight basis of corn syrup was utilized. The resulting powder had an enzyme method assay of about 601, 950 iu/gm. However, after subjecting the spray congealed powder to acetaldehyde, the powder assayed at substantially the same assay, indicating virtually no crosslinking relative to Example I.

### Enzyme Method for Assay of Gelatin Crosslinking

Forty milliliters of a 10 percent ammonium chloride solution were added to a flask containing 300 to 350 milligrams of a spray-congealed powder containing gelatin and vitamin A, as described in Example I above. A few drops of a 10 percent solution of Tween 80 were added to ensure complete wetting of the powder. Then 5 milliliters of a 0.05 percent solution of protease (from Streptomyces griseus) were added. The resulting mixture was then shaken gently for five hours at 37°C after which 40 milliliters of ethanol were added. One hundred milliliters of petroleum ether were then added to the solution, and the solution was extracted. The ether extract was then diluted one hundred fold in 2-propanol. The potency of the vitamin A released per gram of powder was calculated by using the following formula:

$$\frac{ABS @ 326 \ nm \ in \ 1 \ cm \ cell \ x \ 190,000}{powder \ wt \ in \ gm} = iu/gm$$

Examples III, IV, and V demonstrate the effect which different ingredients have on the crosslinking reaction. In particular, these examples show that the acetals and acetaldehyde which were utilized do not create the degree of crosslinking which occurs upon addition of acetaldehyde and a simple alcohol.

### Example III

Five grams of water was added to a small bottle, after which 3.0 grams of ethyl alcohol was added, followed by 3.0 grams of acetaldehyde. The bottle was then filled and the mixture was allowed to react for ten minutes, during which time some heat was evolved. In a beaker, 41.1 grams of water was added, after which the water was heated to 50-60°C. 18.1 grams of corn syrup was them added to the warm water, immediately after which 29.8 grams of gelatin was added to the water/corn syrup solution. After the gelatin was added, the solution was maintained at 50-60°C while the contents of the sealed bottle were added to the warm solution in the beaker. The contents of the beaker were then maintained at 50-60°C for another 15 minutes. The viscosity of the solution in the beaker became noticeably higher as was evidenced by the slowing down of the magnetic stirrer which was used to stir the solution in the beaker. Furthermore, the solution within the beaker turned from a light yellow to a dark amber color during the heating period. After the 15 minute heating period expired, about 6 milliliters of the solution were removed and poured into approximately two millimeter thick slabs. After about one hour the gelled slabs were hung overnight to dry in a flowing stream of room temperature air. The next day a portion of the slab was added to boiling water and it did not dissolve. It was concluded that the ingredients utilized above created a crosslinked gelatin.

### Example IV

An experiment was carried out exactly as described in Example III, except that methyl alcohol was utilized in place of ethyl alcohol. Just as in Example III, the resulting solution turned from a light yellow to a dark amber color during the 15-minute period after the two solutions were added together. Likewise, during the heating period, the viscosity of the solution became noticeably higher as was evidenced by the slowing down of the magnetic stirrer, just as in Example III. The resulting slabs also were insoluble in boiling water. It was therefore concluded that methyl alcohol was the equivalent of ethyl alcohol for the purpose of carrying out the rapid crosslinking reaction.

Example V (Comparative)

46.1 grams of water were added to a beaker and were heated to 50-60°C. While the water was maintained at this temperature, 18.1 grams of corn syrup was added to the water, followed by the addition of 29.8 grams of gelatin. After these ingredients had been added, 6.0 grams of acetaldehyde diethyl acetal were added to the warm beaker. The resulting solution was maintained at 50-60°C for an additional 15-minute period, during which the solution was stirred. The solution maintained a light yellow color throughout the heating period. After the 15-minute heating period, approximately 6 milliliters of the solution was removed and poured into an approximately 2 millimeter thick slab. After about one hour, the gel slab was hung to dry overnight in a flowing stream of room temperature air. The next day a portion of the slab was added to boiling water, and it rapidly dissolved. It was concluded that the acetaldehyde diethyl acetal was ineffective in creating crosslinking of the gelatin.

**Claims**

1. A method of rapidly crosslinking a protein, characterized by combining a protein with a water-miscible alcohol and acetaldehyde so that a moist intimate mixture results.

2. The method as described in claim 1 wherein the intimate mixture comprises protein, alcohol and acetaldehyde within particles which make up a powder.

3. The method as described in claim 2 wherein the powder is made by spray drying or spray congealing.

4. The method as described in anyone of claims 1 to 3 wherein the intimate mixture is made by creating an emulsion.

5. The method as described in anyone of claims 1 to 4 wherein the protein is gelatin.

6. The method as described in anyone of claims 3 to 5 wherein the method comprises the steps of:
a) spray congealing an intimate mixture of gelatin and water-miscible alcohol; and
b) exposing the spray-congealed gelatin and alcohol to acetaldehyde,
and wherein the acetaldehyde is in the vapor state when the spray-congealed gelatin and alcohol are exposed to the acetaldehyde.

7. The method as described in anyone of claims 1 to 6 wherein the water-miscible alcohol is a member selected from the group consisting of sorbitol, propylene glycol, glycerol, mannitol, ethanol, and methanol.

8. The method as described in anyone of claims 1 to 7 wherein the temperature of the intimate mixture is at least 50°C during the addition of aldehyde to the mixture, and the mixture comprises between 3 percent and 25 percent moisture.

9. The method as described in anyone of claims 6 to 8 wherein the acetaldehyde is exposed to the intimate mixture of protein and alcohol while both the acetaldehyde vapor and the intimate mixture of protein and alcohol are under a positive pressure between 34.45 - 206.4 kPa.

10. The method as described in anyone of claims 1 to 9 wherein the mixture additionally comprises a fat-soluble vitamin, particularly vitamin A.

11. The method as described in claim 1 wherein:
a) the alcohol and acetaldehyde are mixed together and allowed to form a reaction product; and
b) the reaction product is added to a solution of the protein so that the protein is rapidly crosslinked.

12. The process as described in claim 11 wherein the protein is gelatin.

13. The process as described in claim 12 wherein an aqueous gelatin solution contains droplets of vitamin A.